# EUROPÄISCHE PATENTSCHRIFT

(11) **EP 1 307 201 B1**
(45) Veröffentlichungstag und Bekanntmachung des Hinweises auf die Patenterteilung: **24.11.2004**
(21) Anmeldenummer: 01969511.3
(22) Anmeldetag: 19.07.2001
(51) Int. Cl.: A61K 31/505, A61P 25/28, A61P 9/10, A61P 25/24, A61P 25/16, A61K 31/52, A61K 31/519

(54) **SELEKTIVE PDE 2-INHIBITOREN ALS ARZNEIMITTEL ZUR VERBESSERUNG DER WAHRNEHMUNG**
SELECTIVE PDE 2 INHIBITORS, USED AS MEDICAMENTS FOR IMPROVING COGNITION
INHIBITEURS SELECTIFS DE PDE 2 COMME MEDICAMENTS AMELIORANT LA PERCEPTION

(30) Priorität: 01.08.2000 DE 10037411; 11.05.2001 DE 10122893
(43) Veröffentlichungstag der Anmeldung: 07.05.2003
(73) Patentinhaber: Bayer HealthCare AG, 51368 Leverkusen (DE)
(72) Erfinder: BÖSS, Frank-Gerhard, 42115 Wuppertal (DE); HENDRIX, Martin, 51519 Odenthal (DE); KÖNIG, Gerhard, BOSTON, MA 02109 (US); NIEWÖHNER, Ulrich, 42929 Wermelskirchen (DE); SCHLEMMER, Karl-Heinz, 42113 Wuppertal (DE); SCHREIBER, Rudy, Menlo Park,CA 94025 (US); VAN DER STAAY, Franz-Josef, 53797 Lohmar (DE); SCHAUSS, Dagmar, 42697 solingen (DE)
(86) Internationale Anmeldenummer: PCT/EP2001/008609
(87) Internationale Veröffentlichungsnummer: WO 2002/009713

(56) Entgegenhaltungen:
- EP-A- 0 771 799
- WO-A-00/12504
- WO-A-00/24745
- WO-A-01/47928
- WO-A-01/47929
- WO-A-01/47934

## Beschreibung

Die Erfindung betrifft die Verwendung von selektiven Phosphodiesterase 2 (PDE 2) - Inhibitoren zur Herstellung von Arzneimitteln zur Verbesserung von Wahrnehmung, Konzentrationsleistung, Lernleisftung und/oder Gedächtnisleistung.

Die zelluläre Aktivierung von Adenylat- bzw. Guanylatzyklasen bewirkt die Zyklisierung von ATP bzw. GTP zu 5'-3' zyklischem Adenosin Monophosphat (cAMP) bzw. 5'-3' zyklischem Guanosin monophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Protein Kinasen. Die von cAMP aktivierte Protein Kinase wird Protein Kinase A (PKA) genannt, die von cGMP aktivierte Protein Kinase wird Protein Kinase G (PKG) genannt (für eine Übersicht: siehe Stryer, L., Biochemistry, 4. Auflage, Freeman, New York, 1995). Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (für eine Übersicht: Kandel et al. in ,Principles ofNeural Science' , 1991, 3. Auflage, Elsevier, Kapitel 28, S. 403 - 408.) Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE Gene beschrieben *(Exp. Opin. Investig. Drugs* **2000,** *9*, 1354-3784). Diese 21 PDE Gene lassen sich aufgrund ihrer Sequenzhomologie in 11 PDE Familien einteilen (Nomenklatur Vorschlag siehe: www.hs.washington.edu.). Einzelne PDE Gene innerhalb einer Familie werden durch Buchstaben unterschieden (z.B. PDE1A und PDE1B). Falls noch unterschiedliche Splice Varianten innerhalb eines Genes vorkommen, wird dies dann durch eine zusätzliche Nununerierung nach dem Buchstaben angegeben (z.B. PDE1A1). Das ursprünglich als "cGMP stimulierte PDE" und später als PDE2 bezeichnete Enzym wurde erstmals 1982 aus Rinderherzen und Rindemebenniere biochemisch isoliert und gereinigt (Martins et al. *J. Biol. Chem.* **1982,** *257*, 1973-1979). Die Besonderheit dieser Phosphodiesterase liegt in ihrer positiven kooperativen Kinetik bzgl. des Substrates cGMP. Es wurde postuliert, dass geringe Mengen von cGMP an die sogenannte cGMP-bindende Domäne binden und dadurch eine Aktivierung des Enzyms bewirken. Hierdurch erhöht sich auch die Affinität der katalytischen Domäne gegenüber cGMP und cAMP (Martins et al. *J. Biol. Chem.* **1982,** 257, 1973-1979). Deswegen kann PDE2 durch geringe Mengen von cGMP beide second messenger-Systeme hydrolysieren und dadurch auch kontrollieren.

Durch cGMP stimulierte PDE's wurden auch in anderen verschiedenen Geweben beschrieben. So u.a., aber nicht ausschließlich, in der Leber (Yamamoto et al. *J. Biol. Chem.* **1983,** *258*, 12526-12533.) und in Thrombozyten (Grant et al. *Thromb Res.* **1990,** *59*, 105-119.). Eine membrangebundene Form der cGMP-stimulierten PDE wurde aus Kaninchenhirn isoliert (Whalin et al. *Biochim. Biophys. Acta.* **1988,** *972,* 79-94.). Kloniert wurde die cDNA von PDE2 erstmals aus Rind und Ratte (Sonnenburg et al. *J.Biol Chem.* **1991,** *266,* 17655-17661.; Tanaka et al. *Second Messengers Phosphoproteins.* **1991,** *13*, 87-98.). Sonnenburg et al. zeigte auch die starke Expression der PDE2-mRNA in der Gehirnrinde (cortex), den Basalganglien sowie im Hippocampus. Die Sequenz der menschlichen Isoform PDE2A3 Sequenz (Gen Bank Acc. No. U67733) wurde von Rosman et al. *Gene.* **1997,** *191*, 89-95. berichtet. Von den untersuchten Geweben wurde hierin die Expression von PDE2A stark in Gehirn und Herz und schwächer in Leber, Skelettmuskel, Niere und Pankreas nachgewiesen.

Als spezifischer Inhibitor der PDE2 wurde bisher nur das erythro-9-(2-Hydroxy-3-nonyl)-adenin (EHNA: IC₅₀=1µM) beschrieben. Allerdings ist EHNA auch ein sehr potenter Adenosin Deaminase Inhibitor (IC₅₀=3nM) wodurch zellbiologische und *in vivo*-pharmakologische Effekte, die mit EHNA erzeugt wurden, nicht eindeutig interpretiert werden können.

Die EP-A-0 771 799, die WO 98/40384 und die WO 00/12504 beschreiben Purinon-, Allopurinol- bzw. Triazolopyrimidinon-Derivate, deren PDE-inhibitorische Wirkung und ihre Eignung zur Behandlung von bestimmten Gefäßerkrankungen.

Überraschenderweise wurde nun gefunden, dass selektive PDE 2-Inhibitoren zur Herstellung von Arzneimitteln zur Verbesserung der Wahrnehmung, Konzentrationsleistung, Lemleistung oder Gedächtnisleistung geeignet sind.

Ein PDE 2-Inhibitor im Sinne der Erfindung ist eine Verbindung, die humane PDE 2 unter den unten angegebenen Bedingungen mit einem IC₅₀ von weniger als 10 µM, bevorzugt weniger als 1 µM, besonders bevorzugt weniger als 0,1 µM hemmt.

Ein selektiver PDE 2-Inhibitor im Sinne der Erfindung ist eine Verbindung, die humane PDE 2 unter den unten angegebenen Bedingungen stärker hemmt als die humanen cAMP-PDEs 3B, 4B und 7B. Bevorzugt ist IC₅₀ (PDE 2)/ IC₅₀ (PDE 3B, 4B oder 7B) kleiner als 0,1.

Besonders eignen sich die selektiven PDE 2-Inhibitoren zur Verbesserung der Wahrnehmung, Konzentrationsleistung, Lernleistung, oder Gedächtnisleistung nach Kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Him-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern- und Gedächtnisproblemen, Alzheimersche Krankheit, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsak-off-Psychose.

Die Erfindung betrifft bevorzugt die erfindungsgemäße Verwendung von PDE 2-Inhibitoren der allgemeinen Formel (I), 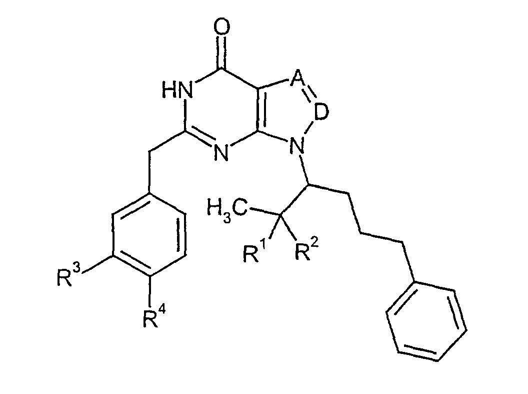 in welcher
- A=D: für N=N, N=CH oder CR⁵=N steht, worin R⁵ Wasserstoff, Methyl, Ethyl oder Methoxy bedeutet,
- R¹ und R²: zusammen mit dem angrenzenden Kohlenstoffatom für Hydroxymethylen oder Carbonyl stehen, und
- R³ und R⁴: unabhängig voneinander für Methyl, Ethyl, Methoxy, Ethoxy oder einen Rest der Formel SO₂NR⁶R⁷ stehen,
worin
- R⁶ und R⁷: unabhängig voneinander Wasserstoff, (C₁-C₆)-Alkyl, (C₃-C₇)-Cycloalkyl bedeuten, oder
- R⁶ und R⁷: zusammen mit dem benachbarten Stickstoffatom einen Azetidin-1-yl-, Pyrrol-1-yl-, Piperid-1-yl-, Azepin-1-yl, 4-Methyl-piperazin-1-yl-oder Morpholin-1-yl-Rest bilden,
oder eines ihrer Salze.

(C₁-C₆)-Alkyl steht im Rahmen der Erfindung für einen geradkettigen oder verzweigten Alkylrest mit 1 bis 6 Kohlenstoffatomen. Bevorzugt ist ein geradkettiger oder verzweigter Alkylrest mit 1 bis 4 Kohlenstoffatomen. Beispielsweise seien genannt: Methyl, Ethyl, n-Propyl, Isopropyl, t-Butyl, n-Pentyl und n-Hexyl.

(C₃-C₇)-Cycloalkyl steht im Rahmen der Erfindung für eine Cycloalkylgruppe mit 3 bis 7 Kohlenstoffatomen. Bevorzugt seien genannt: Cyclopropyl, Cyclobutyl, Cyclopentyl, Cyclohexyl und Cycloheptyl.

Die erfindungsgemäßen Stoffe der allgemeinen Formel (I) können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren, wie beispielsweise Chlorwasserstoffsäure, Bromwasserstoffsäure, Phosphorsäure oder Schwefelsäure, oder Salze mit organischen Carbonoder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die Verbindungen der allgemeinen Formel (I) sind aus der EP-A-0 771 799, der WO 98/40384 und der WO 00/12504 bekannt oder können nach dort beschriebenen Verfahren hergestellt werden. Auf die Offenbarung der EP-A-0 771 799, der WO 98/40384 und der WO 00/12504 wird ausdrücklich Bezug genommen.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. magensaftresistente Überzüge), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im allgemeinen hat es sich als vorteilhaft erwiesen, bei parenteraler Applikation Mengen von etwa 0,001 bis 30 mg/kg, vorzugsweise etwa 0,01 bis 10 mg/kg Körpergewicht zur Erzielung wirksamer Ergebnisse zu verabreichen. Bei oraler Applikation beträgt die Menge etwa 0,01 bis 100 mg/kg, vorzugsweise etwa 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

### PDE-Inhibition

Die cGMP-stimulierbare PDE 2 wird aus Rindermyokard isoliert. Die Ca²⁺-Calinodulin-stimulierbare PDE 1 wird aus Schweineaorta, Schweinehirn oder bevorzugt aus Rinderaorta isoliert. Die cGMP-spezifische PDE 5 wird aus Schweinedünndarm, Schweineaorta, humanen Blutplättchen und bevorzugt aus Rinderaorta gewonnen. Die Reinigung erfolgt durch Anionenaustauschchromatographie an MonoQ^{R} Pharmacia im wesentlichen nach der Methode von Hoey, M; Houslay, M.D., *Biochem. Pharmacol.* **1990**, *40,* 193-202 und Lugman et al. *Biochem. Pharmacol.* ― **1986**, *35*, 1743-1751.

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq [³H]-cAMP oder [³H]-cGMP enthält. Die Endkonzentration der entsprechenden Nucleotide ist 10⁻⁶ mol/l. Die Reaktion wird durch Zugabe des Enzyms gestartet, die Enzymmenge ist so bemessen, dass während der Inkubationszeit von 30 min ca. 50 % des Substrates umgesetzt werden. Um die cGMP stimulierbare PDE 2 zu testen, wird als Substrat [³H]-cAMP verwendet und dem Ansatz 10⁻⁶ mol/l nicht markiertes cGMP zugesetzt. Um die Ca-Calmodulinabhängige PDE 1 zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflussscintillationszähler quantitativ bestimmt. Es wird die Substanzkonzentration gemessen, bei der die Reaktionsgeschwindigkeit um 50 % vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt.

Humane rekombinante PDE2 (Rosman et al. *Gene* **1997** *191*, 89-95), PDE3B (Miki et al. *Genomics* **1996** *36,* 476-485), PDE4B (Bolger et al. *Mol. Cell. Biol.* **1993** *13*, 6558-6571) und PDE7B (Hetman et al. *Proc. Natl. Acad. Sci. U.S.A.* **2000** *97*, 472-476) werden mit Hilfe des pFASTBAC Baculovirus Expressionssystems (GibcoBRL) in Sf9 Zellen exprimiert.

Die Bestimmung der Aktivität der Testsubstanzen an humaner rekombinanter PDE2, PDE3B, PDE4B und PDE7B erfolgt mit dem [³H]cAMP Scintillation Proximity Assay (SPA) Kit (TRKQ7090) von Amersham International (Little Chalfont, England) bzw. an PDE1 und PDE5 mit dem [³H]cGMP Scintillation Proximity Assay (SPA) Kit (TRKQ7100) von Amersham International (Little Chalfont, England).

Testsubstanzen werden in 100 % DMSO gelöst (10 mM), und diese Lösung wird weiter mit H₂O verdünnt (höchste Endkonzentration im Test: 10µM). Zur Vorstimulation der PDE2 wird cGMP beigefügt (Endkonzentration im Test: 10⁻⁶ M). Das Enzym wird in PDE Puffer (20mM TRIS/HCl, 5mM MgCl₂, 0,1 mg/ml Albumin, pH 7,5) verdünnt. In einer 96-Loch Platte (Wallac, 1450-401) werden pro Loch folgende Volumina pipettiert: 10 µl Substanzlösung (beim 100% Wert 10 µl H₂O), 10 µl cGMP (10⁻⁵ M), 70 µl [³H]-cAMP Testgemisch (siehe Kit), 10 µl Enzym (beim 0-Wert kein Enzym, stattdessen + 10µl H₂O) zum Start der Reaktion. Nach 15 min Inkubation bei 30°C wird die Reaktion mit 50 µl SPA-Beadlösung (siehe Kit) gestoppt, die Platte mit einer Folie verschlossen und 30 Sekunden geschüttelt. Nach dem Absetzen der Beads (ca. 15 min) wird die Platte im beta-counter gemessen.

Für die Messung der PDE1 wird Calmodulin 10⁻⁷ M und CaCl₂ 1µM zum Reaktionsansatz zugegeben. Die PDE5 wird mit dem [³H] cGMP SPA Assay gemessen. Die PDE3B, PDE4B und PDE7B wird mit dem [³H] cAMP scintillation proximity assay gemessen.

Das verwendete Ausführungsbeispiel 1, 6-(3,4-Dimethoxy-benzyl)-1-[1-(1-hydroxyethyl)-4-phenyl-butyl]-3-methyl-1,5-dihydro-pyrazolo[3,4-*d*]pyrimidin-4-on, entspricht dem Beispiel 36 in der WO 98/40384 und wurde nach dem dort beschriebenen Verfahren hergestellt.

Inhibition von PDE-Isoenzymen durch Beispiel 1:

| Isoenzym | Species | IC₅₀ [nM] |
|---|---|---|
| PDE 1 | Rind | 200 |
| PDE2 | Rind | 7 |
| PDE2 | human | 6 |
| PDE3B | human | > 4000 |
| PDE4B | human | 2900 |
| PDE5 | human | 300 |
| PDE7B | human | 1600 |

### Erhöhung der intrazellulären neuronalen cGMP-Konzentration in Zellkulturen

PDE 2-Inhibitoren erhöhen die intrazelluläre neuronale cGMP Konzentration nach Vorstimulierung der Guanylatzyklase mit 10⁻⁴ M Natriumnitroprussid (SNP) in primären Maushimzellkulturen.

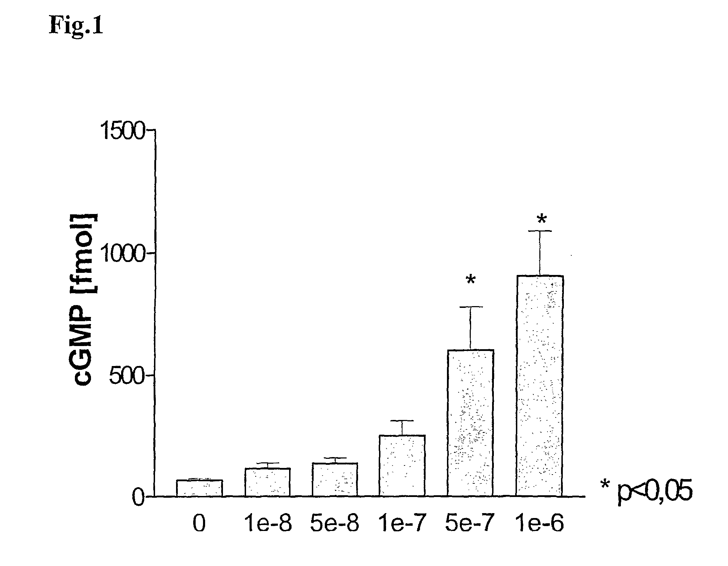

Mausembryonen wurden dekapitiert, die Köpfe in Präparationsschalen überführt. Die Kopfhaut und Schädeldecke wurde entfernt, und die freipräparierten Gehirne wurden in eine weitere Petrischale überführt. Mithilfe eines Binokulars und zweier Pinzetten wurde das Großhirn (Cortex) isoliert und mit Eis auf 4°C gekühlt. Diese Präparation und die Vereinzelung der kortikalen Neuronen wurden dann nach einem Standardprotokoll mit dem Papain Dissociationssystem (Worthington Biochemical Corporation, Lakewood, New Jersey 08701, USA) durchgeführt (Huettner et al. *J*. *Neurosci.* **1986**, *6*, 3044-3060.). Die mechanisch vereinzelten Neuronen wurden zu 150.000 Zellen/Loch in 200 µl Neurobasalmedium/Loch (Neurobasal; Gibco/BRL; 2mM L-Glutamin; in Anwesenheit von Penicillin/Streptomycin) 7 Tage in 96 Lochplatten (mit Poly-D Lysin 100µg/ml für 20 min vorbehandelt) unter Standard Bedingungen kultiviert (37°C, 5%CO₂). Nach 7 Tagen wurde das Medium abgenommen und die Zellen mit HBS Puffer (Gibco/BRL) gewaschen. Anschließend wurden je 100µl SNP-Lösung und 100µL von Beispiel 1 (zuvor in 100% DMSO gelöst: 10 mM) in HBS auf die Zellen gegeben, so dass die Endkonzentration von SNP 100 mM und die von Beispiel 1 so lag wie in Figur 1 angegeben und bei 37°C für 20min inkubiert. Danach wurden die Zellen in 200µl Lysispuffer (cGMP Kit code RPN 226; von Amersham Pharmacia Biotech.) lysiert und die cGMP Konzentration nach den Angaben des Herstellers gemessen. Alle Messungen wurden in Triplicaten durchgeführt. Die Statistische Auswertung erfolgte mit Prism Software Version 2.0 (GraphPad Software Inc., San Diego, CA USA).

Bei paralleler Inkubation von Neuronen mit SNP (einem Stimulator der Guanylatzyklase) und Beispiel 1 zeigte sich bereits ab einer Konzentration von 100nM eine deutliche Erhöhung des intrazellulären cGMP Spiegels (Fig.1 ).

**Fig. 1:** Intrazelluläre cGMP-Konzentration in primären Maus (E18)-Kortexkulturen (Ordinate) nach Behandlung mit SNP und Beispiel 1 (Abszisse)

Zellen wurden mit 100mM SNP (0) oder mit 100mM SNP und Beispiel 1 (1x10⁻⁸ M; 5x10⁻⁸ M; 1x10⁻⁷ M; 5x10⁻⁷ M; 1x10⁻⁶ M) für 20 min behandelt. Danach wurde der intrazelluläre cGMP Spiegel gemessen.

### Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wurde wie beschrieben durchgeführt (Blokland et al. *NeuroReport* **1998,** *9*, 4205-4208; Ennaceur, A., Delacour, J., *Behav. Brain Res.* **1988,** *31*, 47-59; Ennaceur, A., Meliani, K.,. *Psychopharmacology* **1992,** *109,* 321-330; Prickaerts, et al. *Eur. J. Pharmacol.* **1997**, *337,* 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einem Intervall von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekter gleichstark inspektieren. Die Gabe einer Substanz mit lern- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

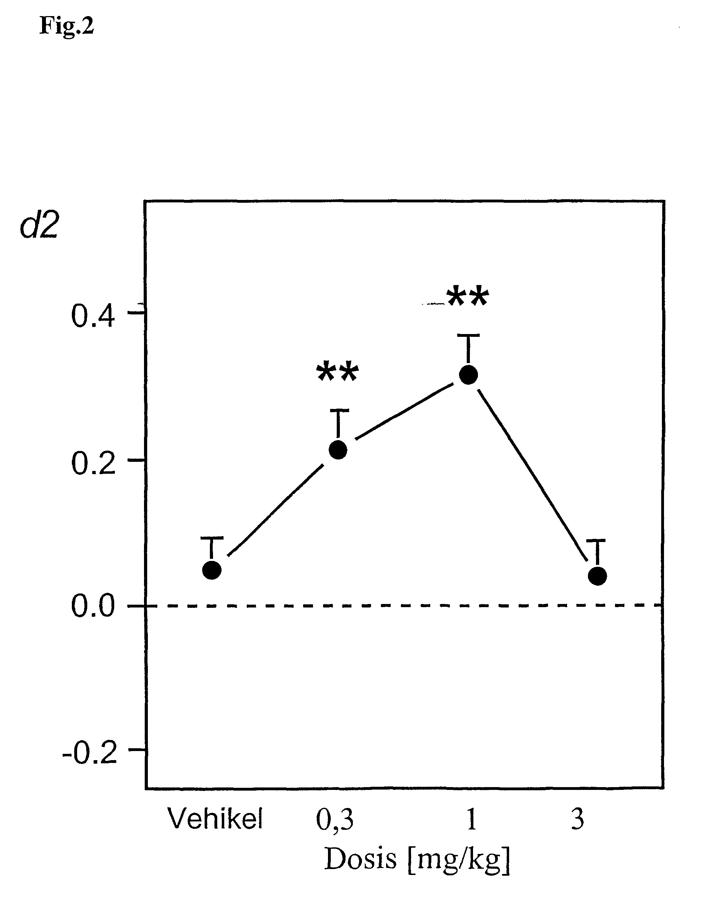

Die Effekte von Beispiel 1 auf die Objekt-Wiedererkennung von Ratten 24 Stunden nach dem ersten Durchgang wurden untersucht. Die Tiere erhielten oral Tylose alleine, oder Beispiel 1 in den Dosierungen 0,3, 1,0 oder 3,0 mg/kg Körpergewicht, suspendiert in Tylose, unmittelbar im Anschluss an den ersten Durchgang mit zwei identischen Objekten. Jeweils 24 Stunden später folgte der zweite Durchgang. Nach einer Auswaschperiode von 2 oder 3 Tagen wurde in denselben Ratten eine neue Dosierung von Beispiel 1 getestet, bis die Gedächtnisleistung aller Ratten zweimal in allen Dosierungen erfasst worden war. Alle Tiere dienten also als eigene Kontrolle. Die Resultate dieser Studie sind in Fig. 2 wiedergegeben. Überraschenderweise war die Gedächtnisleistung im zweiten Durchgang nach Behandlung mit 0,3 und 1,0 mg/kg von Beispiel 1 gegenüber der Kontrollbedingung (Behandlung mit Tylose alleine) verbessert. Der Diskriminationsindex war größer als Null und wich von dem in der Kontrollbedingung erreichten Diskriminationsindex ab.

### Die Ergebnisse dieses Tests sind in Fig.2 dargestellt:

**FIG. 2**: Wirkung von Beispiel 1 auf den Diskriminationsindex (d2) im Objekt-Wiedererkennungstest (Durchschnittswerte + S.E.M). Vehikel Behandlung war mit 1% Tylose. Die mit Substanz behandelten Tiere wurden mit 0,3 mg/kg, 1mg/kg bzw. 3mg/kg behandelt. Statistische Auswertung: ***P* < 0.01.

## Patentansprüche

1. Verwendung von selektiven PDE 2-Inhibitoren zur Herstellung von Arzneimitteln zur Verbesserung der Wahrnehmung, Konzentrationsleistung, Lernleistung und/oder Gedächtnisleistung.

2. Verwendung nach Anspruch 1 zur Prophylaxe und/oder Behandlung von Störungen der Wahrnehmung, Konzentrationsleistung, Lernleistung und/oder Gedächtnisleistung.

3. Verwendung nach Anspruch 2, wobei die Störung eine Folge von Demenz ist.

4. Verwendung nach Anspruch 2, wobei die Störung eine Folge von Schlaganfall oder Schädel-Him-Trauma ist.

5. Verwendung nach Anspruch 2, wobei die Störung eine Folge der Alzheimerschen Krankheit ist.

6. Verwendung nach Anspruch 2, wobei die Störung eine Folge der Parkinsonschen Krankheit ist.

7. Verwendung nach Anspruch 2, wobei die Störung eine Folge von Depression ist.

8. Verwendung nach Anspruch 2, wobei die Störung eine Folge der Demenz mit Frontallappendegeneration ist.

9. Verwendung nach einem der Ansprüche 1 bis 8, wobei der selektive PDE 2-Inhibitor eine Verbindung der allgemeinen Formel (I), 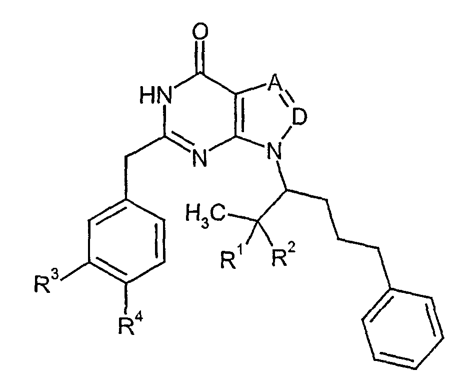 in welcher
A=D für N=N, N=CH oder CR⁵=N steht, worin R⁵ Wasserstoff, Methyl, Ethyl oder Methoxy bedeutet,
R¹ und R² zusammen mit dem angrenzenden Kohlenstoffatom für Hydroxymethylen oder Carbonyl stehen, und
R³ und R⁴ unabhängig voneinander für Methyl, Ethyl, Methoxy, Ethoxy oder einen Rest der Formel SO₂NR⁶R⁷ stehen,
worin
R⁶ und R⁷ unabhängig voneinander Wasserstoff, (C₁-C₆)-Alkyl, (C₃-C₇)-Cycloalkyl bedeuten, oder
R⁶ und R⁷ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
ist, oder eines ihrer Salze.

## Claims

1. Use of selective PDE 2 inhibitors for producing pharmaceuticals for improving perception, concentration, learning and/or memory.

2. Use according to Claim 1 for the prophylaxis and/or treatment of disorders of perception, concentration, learning and/or memory.

3. Use according to Claim 2, where the disorder is a result of dementia.

4. Use according to Claim 2, where the disorder is a result of stroke or craniocerebral trauma.

5. Use according to Claim 2, where the disorder is a result of Alzheimer's disease.

6. Use according to Claim 2, where the disorder is a result of Parkinson's disease.

7. Use according to Claim 2, where the disorder is a result of depression.

8. Use according to Claim 2, where the disorder is a result of dementia with frontal lobe degeneration.

9. Use according to any of Claims 1 to 8, where the selective PDE 2 inhibitor is a compound of the general formula (1) 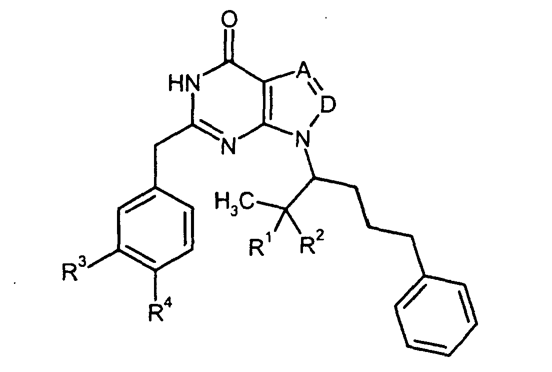 in which
A=D represents N=N, N=CH or CR⁵=N, in which R⁵ denotes hydrogen, methyl, ethyl or methoxy,
R¹ and R² represent, together with the adjacent carbon atom, hydroxymethylene or carbonyl, and
R³ and R⁴ represent independently of one another methyl, ethyl, methoxy, ethoxy or a radical of the formula SO₂NR⁶R⁷,
in which
R⁶ and R⁷ denote, independently of one another, hydrogen, C₁-C₆-alkyl, C₃-C₇-cycloalkyl, or
R⁶ and R⁷ form, together with the adjacent nitrogen atom, an azetidin-1-yl, pyrrol-1-yl, piperid-1-yl, azepin-1-yl, 4-methylpiperazin-1-yl or morpholin-1-yl radical,
or one of its salts.

## Revendications

1. Utilisation d'inhibiteurs sélectifs de PDE 2 pour la préparation de médicaments destinés à améliorer la perception, la capacité de concentration, la capacité d'apprentissage et/ou la capacité de mémoire.

2. Utilisation suivant la revendication 1, pour la prophylaxie et/ou le traitement de troubles de la perception, de la capacité de concentration, de la capacité d'apprentissage et/ou de la capacité de mémoire.

3. Utilisation suivant la revendication 2, dans laquelle le trouble est une conséquence de la démence.

4. Utilisation suivant la revendication 2, dans laquelle le trouble est une séquelle d'apoplexie cérébrale ou de traumatisme cranio-cérébral.

5. Utilisation suivant la revendication 2, dans laquelle le trouble est une conséquence de la maladie d'Alzheimer.

6. Utilisation suivant la revendication 2, dans laquelle le trouble est une conséquence de la maladie de Parkinson.

7. Utilisation suivant la revendication 2, dans laquelle le trouble fait suite à une dépression.

8. Utilisation suivant la revendication 2, dans laquelle le trouble est une conséquence de la démence avec dégénérescence du lobe frontal.

9. Utilisation suivant l'une des revendications 1 à 8, dans laquelle l'inhibiteur sélectif de PDE 2 est un composé de formule générale (I), 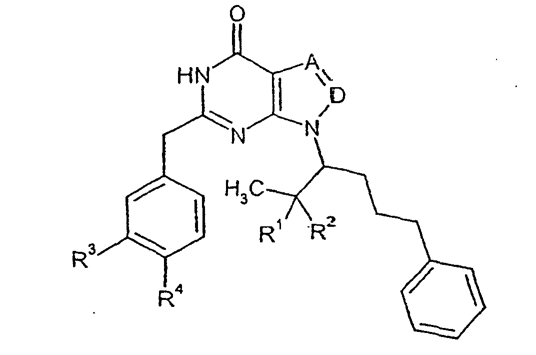 dans laquelle
A=D représente N=N, N=CH ou CR⁵=N, où R⁵ représente l'hydrogène, un reste méthyle, éthyle ou méthoxy,
R¹ et R² forment conjointement avec l'atome de carbone contigu un groupe hydroxyméthylène ou carbonyle, et
R³ et R⁴ représentent, indépendamment l'un de l'autre, un reste méthyle, éthyle, méthoxy, éthoxy ou un reste de formule SO₂NR⁶R⁷,
dans laquelle
R⁶ et R⁷ représentent indépendamment l'un de l'autre l'hydrogène, un radical alkyle en C₁ à C₆, cycloalkyle en C₃ à C₇ ou bien
R⁶ et R⁷ forment conjointement avec l'atome de carbone voisin un reste azétidine-1-yle, pyrrole-1-yle, pipéridine-1-yle, azépine-1-yle, 4-méthylpipérazine-1-yle ou morpholine-1-yle,
ou l'un de ses sels.
